# FASCICULE DE BREVET EUROPEEN

(11) **EP 1 562 549 B1**
(45) Date de publication et mention de la délivrance du brevet: **08.03.2017**
(21) Numéro de dépôt: 03782522.1
(22) Date de dépôt: 06.11.2003
(51) Int. Cl.: A61K 9/00, A61K 47/36, A61K 47/42, A23K 50/00

(54) **SUPPORT APPETENT POUR MEDICAMENT DESTINE A UN ANIMAL**
APPETITANREGENDER TRÄGER FÜR TIERMEDIZIN
PALATABLE CARRIER FOR MEDICINE FOR AN ANIMAL

(30) Priorité: 08.11.2002 FR 0214032
(43) Date de publication de la demande: 17.08.2005
(73) Titulaire: Maloisel, Jean-Pierre Arthur Henri, 31000 Toulouse (FR)
(72) Inventeur: Maloisel, Jean-Pierre Arthur Henri, 31000 Toulouse (FR)
(74) Mandataire: Lavoix
(86) Numéro de dépôt international: PCT/FR2003/003318
(87) Numéro de publication internationale: WO 2004/043427

(56) Documents cités:
- EP-A- 0 320 320
- EP-A- 0 574 301
- DE-A- 19 853 729
- US-A- 5 674 515
- US-A- 5 853 757

## Description

L'invention concerne un support appétent pour une substance active destinée à un animal, en particulier pour les chiens, les chats, les chevaux, les porcs et les ruminants.

L'administration de substances actives par voie orale, en particulier de médicaments, aux animaux est souvent difficile du fait du mauvais goût de certaines de ces substances et du sens très développé de l'odorat et du goût de ces animaux.

En cas de refus d'absorption, les chances de succès du traitement vétérinaire peuvent chuter dramatiquement.

Il existe donc un besoin pour un conditionnement des substances actives apte à déjouer la vigilance des animaux, en particulier des chiens, des chats, des chevaux, des porcs et des ruminants..

On connaît de EP 0 320 320 un comprimé comportant un noyau, contenant les substances actives, et une matrice appétente enrobant complètement ce noyau. Un tel comprimé est coûteux à fabriquer, en particulier du fait de l'étape d'enrobage. Il n'est donc pas envisageable d'enrober extemporanément et à l'unité des médicaments livrés dépourvus de matrice appétente.

On connaît de EP 0 574 301 une pastille constituée d'une matrice en un matériau appétent comportant un logement de forme sensiblement complémentaire à celle du comprimé à faire avaler au chien. Le comprimé est bloqué en force dans le logement puis la pastille est présentée au chien de manière à cacher le comprimé.

Une telle pastille présente plusieurs inconvénients.

En premier lieu, la pastille est fabriquée avec son logement. Il n'est plus possible ensuite de modifier la forme de ce dernier, en particulier pour l'adapter à des comprimés de formes différentes.

En second lieu, si l'animal mord dans la pastille, il peut la rompre et provoquer le détachement de la substance active. Il peut alors sentir cette substance active et la recracher.

En dernier lieu, il est impossible avec une telle pastille d'insérer simultanément plusieurs comprimés de même taille, ou d'insérer des gélules.

Il existe donc un besoin pour un support de substances actives ne présentant pas les inconvénients ci-dessus.

Le but de la présente invention est de fournir un tel support.

Selon l'invention, on atteint ce but au moyen d'un support appétent pour une substance active destinée à un animal, comportant une matrice en un matériau comestible, remarquable en ce que ladite matrice comporte au moins une surface collante suffisamment adhésive pour fixer ladite substance active par simple contact de ladite substance active avec ladite surface collante.

Un tel support appétent permet donc de fixer la substance active, notamment un médicament, en la disposant simplement en contact avec la surface collante de la matrice.

Aucun procédé complexe d'enrobage n'est donc nécessaire.

De plus, la fixation par collage autorise l'utilisation du support selon l'invention pour des substances actives préparées par exemple sous forme de comprimés, de tablettes, de gélules ou de poudres, avec des formes quelconques, par exemple sensiblement cylindriques, sphériques ou discoïdales.

Selon d'autres caractéristiques préférentielles du procédé suivant l'invention,
- ladite surface collante est suffisamment adhésive pour que ladite substance active fixée ne puisse en être détachée sans déchirement, au moins partiel, de ladite matrice ;
- ladite matrice comporte au moins deux surfaces collées l'une à l'autre et conformées de manière à pouvoir être écartées l'une de l'autre, à la main, découvrant ainsi ladite surface collante ;
- ladite matrice comporte au moins une masse gluante pouvant être ouverte de manière à exposer ladite surface collante ;
- ladite matrice est déchirable à la main de manière ouvrir ladite masse gluante ;
- ladite matrice est déformable à la main ;
- ladite matrice renferme une substance appétente ;
- ledit support comporte une couche d'une substance appétente recouvrant, au moins en partie, ladite matrice ;
- ladite substance appétente est une poudre de foie ;
- ladite matrice est en une matière de type guimauve,
- ladite matrice est en une matière de type pâte de viande, d'abats ou de viscères, un agent collant étant incorporé à ladite matrice;
- ladite matrice est une matière appétente pour ledit animal ;
- ladite substance active est destinée à un chien, un chat, un cheval, un porc ou un ruminant.

L'invention concerne également un procédé de conditionnement d'une substance active destinée à un animal au moyen d'un support appétent comportant une matrice en un matériau comestible. Ce procédé est remarquable en ce qu'il comporte une étape de collage de ladite substance active sur une surface collante de ladite matrice.

Selon d'autres caractéristiques préférentielles de l'invention,
- préalablement audit collage, on ouvre ladite matrice de manière à exposer ladite surface collante vers l'extérieur dudit support ;
- on procède à ladite ouverture par déchirement de ladite matrice ;
- après ledit collage, on referme ladite matrice de manière à recouvrir, au moins partiellement, ladite substance active ;
- on referme ladite matrice de manière à retenir ladite substance active.

D'autres caractéristiques et avantages de la présente invention apparaîtront à la lecture de la description qui va suivre et à l'examen du dessin annexé dans lequel :
- les figures 1 à 3 représentent des vues en coupes transversales de supports selon l'invention, selon différents modes de réalisation ;
- les figures 4 à 8 représentent des vues en coupes transversales des supports représentés sur les figures 1 à 3, après collage d'un comprimé sur ces supports.

Sur les différentes figures, des références identiques ont été utilisées pour désigner des objets identiques ou analogues.

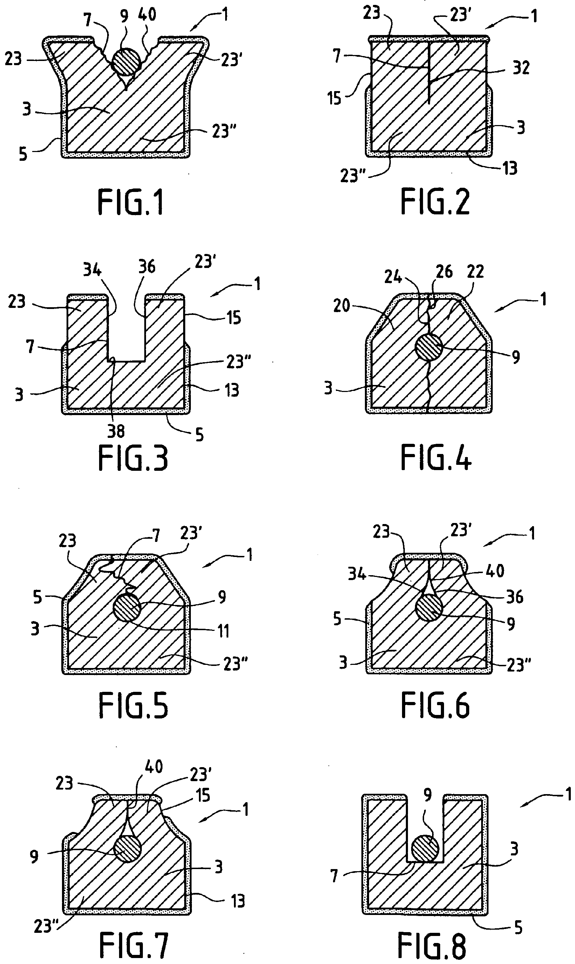

Sur les figures 1 à 8, le support 1 comporte une matrice 3 enrobée au moins en partie d'une couche 5 en un matériau appétent ou « exhausteur d'appétence ».

Comme on le verra plus en détail dans la suite de la description, selon l'invention, la matrice 3 comporte au moins une surface collante 7 destinée à fixer une substance active préparée, par exemple, sous la forme d'un comprimé sensiblement sphérique 9.

La surface collante 7 est suffisamment adhésive pour que la substance active ne puisse être détachée de la matrice 3 sans que la matrice 3 ne laisse un dépôt de matière sur la substance active. Ce dépôt peut être par exemple un dépôt poisseux, notamment de liquide sucré, formant un film, éventuellement discontinu. De préférence, la matrice 3 se déchire localement à l'endroit du détachement de la substance active, la fraction déchirée de la matrice 3 restant collée à la substance active détachée.

Avantageusement, en cas de dislocation de la matrice 3, par exemple si la matrice 3 est réduite en morceaux dans la gueule de l'animal, l'effet de masquage du goût de la substance active par la matrice 3 persiste donc. L'adhésivité de la surface collante 7 est peut être ajustée en fonction de la nature de la substance active 9 qu'elle est destinée à recevoir, par exemple en modifiant la composition de la matrice 3 selon des techniques connues de l'homme du métier.

Il convient de donner à l'adjectif « collant » son sens commun, à savoir une capacité à maintenir par adhérence durable la substance active.

De préférence, la surface 7 est collante au toucher de manière à retenir les granulés solides de type comprimé, tablette ou gélule pendant toute la manipulation du support précédent l'ingestion par l'animal.

La forme de la matrice 3 est par exemple cubique, mais peut être quelconque, pourvu qu'elle soit adaptée à l'ingestion par l'animal à qui est destiné le comprimé 9.

La matrice 3 est de préférence une matière molle à température ambiante. Il est ainsi possible de la déformer à la main pour augmenter la surface de contact entre le comprimé 9 et la surface collante 7. De préférence, la surface de contact couvre sensiblement toute la surface extérieure 11 du comprimé 9 de manière à englober ce dernier.

La matrice 3 est de préférence une matière à déformation plastique, c'est-à-dire qu'elle conserve sensiblement la forme qui lui est donnée, par exemple une gomme ou un gel.

La matrice 3 peut être par exemple une pâte de guimauve comportant classiquement du sucre, du sirop de glucose et de la gélatine. De préférence, le sucre est remplacé par des polyols ou tout autre substitut du sucre connu.

Une composition possible de la guimauve est la suivante :

| | |
|---|---|
| - Sucre: | 37% |
| - Sirop de glucose : | 38% |
| - Dextrose : | 3,5% |
| - Gélatine : | 3,4% |
| - Sucre inverti : | 7% |
| - Eau: | 11,1% |

La matrice peut également être en une matière de type pâte de viande, d'abats ou de viscères. Pour que ces matières puissent présenter une surface collante, un ou plusieurs agents collants, dont au moins du sirop de glucose, sont mélangés dans ladite pâte de viande, d'abats ou de viscères.

Les autres agents collants comprennent en particulier la gélatine et ses coproduits, les hydrocolloides, les gommes, les graisses, par exemple le suif, l'amidon natif ou modifié, des protéines texturées de manière à augmenter leur capacité à retenir l'eau et les matières grasses, les farines prégélatinisées.

Les protéines texturées, par exemple des caséinates ou des protéines de soja, peuvent être classiquement obtenues par cuisson-extrusion.

Un agent collant est le Progel® qui est un coproduit de la gélatine de porc commercialisé par la société SKW (Angoulème-France). Une quantité de Progel® supérieure à 25 %, de préférence supérieure à 30 %, en pourcentage en poids de la matrice, est avantageuse.

La quantité d'agents collants est de préférence supérieure à 10 %, de préférence supérieure à 30 %, de préférence encore supérieure à 45 %, en pourcentage en poids de la matrice.

De préférence, la quantité de viande, d'abats ou de viscères dans la pâte de viande, d'abats ou de viscères, respectivement, est supérieure à 20 %, en pourcentage en poids de la matrice.

Une composition possible de la pâte de viscères est la suivante :

| | |
|---|---|
| - Amidon prégélatinisé : | 28% |
| - Farine de volailles : | 35% |
| - Arôme foie de boeuf : | 1,7% |
| - Conservateurs : | 1% |
| - Sirop de glucose : | 5% |
| - Colorant jaune : | 0,3% |
| - Hydrocolloides : | 2% |
| - Eau: | 27% |

Un autre exemple de composition possible de la pâte de viande est la suivante :

| | |
|---|---|
| - Progel ® : | 30,25% |
| - Amidon prégélatinisé : | 23% |
| - Farine de volailles : | 23% |
| - Glucose: | 15% |
| - Exhausteur d'appétence : | 3% |
| - Amidon de tapioca : | 4% |
| - Conservateurs : | 0,3% |
| - Sorbate : | 0,7% |
| - Colorant : | 0,5% |
| - Dioxyde de titane : | 0,25% |

Cette composition est celle d'une matrice d'un produit de la Demanderesse, appelé Easypill®, qui s'est avérée particulièrement collante.

De préférence, la matrice comporte entre 20 et 35% en poids d'amidon prégélatinisé et entre 20 et 35% en poids de farine de volailles.

La matrice 3 peut être neutre pour l'animal, c'est-à-dire ne présenter sensiblement aucun goût. De préférence cependant, la matrice est choisie en une matière appétente, déterminée en fonction de l'animal auquel est destiné le support 1.

L'exhausteur d'appétence peut être par exemple du foie ou un autre viscère déshydraté, de la farine de viande, de la caséine, de la levure de bière, un concentré de protéine de poisson, ou un mélange de ces différents exhausteurs d'appétence.

Pour les supports destinés aux chevaux, l'exhausteur d'appétence est de préférence un arôme de pomme, de carotte, de vanille ou de cannelle.

L'exhausteur d'appétence peut se présenter sous la forme d'une poudre ou d'un liquide.

Quant l'exhausteur d'appétence se présente sous la forme d'une poudre, le caractère collant de la guimauve permet avantageusement, non seulement de fixer le comprimé 9, mais aussi de fixer la poudre de l'exhausteur d'appétence.

L'exhausteur d'appétence en poudre peut également être dispersé en phase liquide avant son application sur la matrice 3.

Des essais ont montré que la poudre de foie, seule ou en mélange avec d'autres exhausteurs, était particulièrement préférable.

On pourra par exemple utiliser l'exhausteur d'appétence D'TECH 8P-MP003 Super Prémium commercialisé par la Société SPF (France).

L'exhausteur d'appétence peut être incorporé au sein de la matrice 3, par exemple en ajoutant une poudre de ce facteur d'appétence lors de la fabrication de la matrice 3.

De préférence, l'exhausteur d'appétence est disposé sous la forme d'une couche 5 recouvrant au moins en partie une surface extérieure 13 de la matrice 3. De préférence, comme représenté sur les figures 2, 3, 6 et 7, des zones 15 non couvertes par la poudre d'exhausteur de goût sont prévues pour permettre la manipulation du support 1 sans contact des doigts avec l'exhausteur de goût. Ce dernier peut en effet dégager une odeur désagréable pour l'homme.

Un emballage, non représenté, peut également être conçu pour faciliter la manipulation du support 1 sans contact avec l'exhausteur de goût.

On se reporte à présent aux figures 1 et 4.

Le support 1 est fourni sous la forme d'un cube sensiblement homogène que l'utilisateur déchire en deux morceaux 20 et 22.

Le support 1 est représenté sur la figure 1 dans un état intermédiaire entre son état initial sensiblement cubique et son état déchiré en deux morceaux 20 et 22.

La matrice 3 comporte, dans cet état intermédiaire, deux extrémités 23 et 23' reliées par une base commune 23".

La matrice 3 constitue une masse de matière gluante. En ouvrant cette masse gluante, l'utilisateur expose donc une surface collante 7 formée des surfaces 24 et 26 des morceaux 20 et 22, respectivement, délimitant la déchirure.

Avantageusement, la couche 5 d'exhausteur d'appétence et la forme massive du cube protègent la matrice 3 du dessèchement. La durée de vie du support 1 en est augmentée.

De préférence, après avoir déchiré le support 1 et disposé le comprimé 9 sur une des surfaces collantes 24 et 26, l'utilisateur dispose les morceaux 20 et 22 du support 1 déchiré de manière que les surfaces collantes 24 et 26 soient sensiblement l'une en face de l'autre. Il exerce ensuite une action de manière presser l'un contre l'autre ces morceaux de façon à les faire adhérer l'un à l'autre.

Avantageusement, les surfaces collantes 24 et 26 ne servent donc pas uniquement à fixer le comprimé 9 mais servent également à reconstituer le cube initial après collage du comprimé 9. Les doigts de l'utilisateur qui manipulent le support 1 ne sont dès lors plus exposés à aucune surface collante et n'encourent donc plus le risque de devenir poisseux.

De préférence, les surfaces collantes 24 et 26 sont suffisamment adhésives pour que, après qu'elles ont été apposées l'une sur l'autre, elles ne se détachent plus spontanément l'une de l'autre. Par « spontanément », on entend « sous le seul effet de l'élasticité de la matrice », c'est-à-dire sans intervention de l'utilisateur.

De préférence encore, les surfaces collantes 24 et 26 sont suffisamment adhésives pour ne pas se détacher non plus lorsque l'utilisateur ne cherche pas à les détacher l'une de l'autre, en particulier lorsqu'il manipule le support fermé pour le donner à l'animal.

De préférence, comme représenté sur la figure 5, l'utilisateur ne déchire pas totalement le support 1. Avantageusement, la disposition des surfaces 24 et 26 l'une face à l'autre en est facilitée et plus précise.

De préférence, la déchirure est suffisamment large pour permettre l'insertion complète du comprimé 9. Avantageusement, le support 1 selon l'invention permet ainsi d'encapsuler, d'enrober ou d'englober totalement le comprimé 9 après reconstitution du cube initial. Aucune odeur répulsive ou aucun goût désagréable ne peut donc plus se dégager du comprimé 9 inséré dans le support 1.

De préférence, comme représenté sur la figure 2, le support 1 est pourvu d'une fente 32 débouchant sur au moins trois des faces du support 1. Avantageusement, l'ouverture du support 1 en est facilitée.

Par « ouverture », on entend l'exposition vers l'extérieur de volumes de la matrice 3 initialement au sein de la matrice 3.

La forme plane des surfaces 34 et 36 bordant la fente 32 améliore avantageusement le contact avec le comprimé 9 et le contact mutuel de ces surfaces après fermeture du support 1.

Par « fermeture », on entend l'opération de manipulation du support 1 en vue d'encapsuler ou d'englober totalement le comprimé 9 dans la matière constituant la matrice 3, après sa fixation sur la matrice 3. Les figures 4 à 7 représentent ainsi des supports 1 dans des positions fermées.

Dans une variante de l'invention non représentée, la fente 32 peut être remplacée par une simple incision ne débouchant que sur une seule des faces du support 1.

La fente 32 peut également être remplacée par une section, de part en part, du support 1.

Comme représenté sur la figure 3, la fente 32 peut encore être remplacée par une saignée 42 comportant deux faces latérales 34 et 36, et une face 38 formant un fond. Au moins une des faces de la saignée est collante.

De préférence, les 3 faces sont collantes de manière à pouvoir fermer le support 1 par collage des faces latérales 34 et 36 l'une sur l'autre après collage du comprimé 9 au fond de la saignée 42, comme représenté sur la figure 7.

La fermeture du support 1 est toujours optionnelle. En particulier, le médicament peut être donné à l'animal simplement collé et caché au fond de la saignée 42, comme représenté sur la figure 8. Dans ce cas, il n'est pas nécessaire que la matrice 3 soit déformable.

Dans une variante de l'invention, la saignée 42 peut être remplacée par un trou, éventuellement débouchant.

La fabrication du support 1 selon l'invention représenté sur la figure 1 est particulièrement simple et bon marché. Pour cette fabrication, le matériel et les techniques couramment utilisées pour la fabrication des confiseries à base de guimauve ou pour la fabrication d'aliments pour animaux, dits "Pet-Food", peuvent être mis en oeuvre.

Une pâte de guimauve est découpée ou moulée en cubes, puis ces cubes sont recouverts de poudre de foie. Cette poudre peut être pulvérisée sur la guimauve collante. La guimauve peut aussi être roulée dans cette poudre par exemple au moyen d'une turbine ou "huileuse" ou « sucreuse ». Les cubes peuvent également être enduits d'un revêtement liquide incorporant la poudre de foie, puis mis à sécher.

L'exhausteur d'appétence représente, de préférence, entre 1 et 10% en poids du support.

Selon les différentes variantes décrites ci-dessus, les cubes recouverts de l'exhausteur d'appétence sont alors fendus, percés ou rainurés de manière à prédéfinir des surfaces collantes. Les fentes, incisions, trous, et saignées peuvent également être obtenues lors de la fabrication des cubes. Il faudra alors veiller à ce que le dépôt de l'exhausteur d'appétence n'ôte leur caractère collant aux surfaces destinées à fixer la substance active.

De préférence, les cubes enrobés sont emballés individuellement afin de limiter leur dessèchement et faciliter leur manipulation. Dans le cas des supports pourvus d'une fente, d'une incision, d'un trou ou d'une saignée, il est préférable que l'emballage puisse être retiré partiellement en découvrant une surface collante 7.

Pour procéder au conditionnement du comprimé 9, l'utilisateur déballe au moins partiellement un support 1 selon l'invention.

Si la surface collante 7 n'est pas immédiatement exposée lors du déballage du support 1 (figures 1, 2, 4, 5 et 6), l'utilisateur ouvre la matrice 3 de manière à découvrir une surface collante 7. Cette ouverture peut résulter d'un déchirement, au moins partiel de la matrice 3 (figures 1, 4 et 5) ou d'un écartement de deux parties de la matrice 3 de manière à élargir une fente 32 ménagée dans le support (figures 2 et 5).

Puis l'utilisateur dépose le comprimé 9 sur la surface collante 7. La mise en contact du comprimé avec le comprimé 9 suffit à fixer ce dernier.

De préférence, l'utilisateur presse légèrement, de préférence en appliquant dessus une force d'au moins 0,05 N, le comprimé 9 sur la surface collante 7. Avantageusement, la pression sur le comprimé 9 permet de le mettre en contact intime avec la surface collante 7, et ainsi d'améliorer la qualité de la fixation et d'éviter tout détachement du comprimé 9 pendant la manipulation du support.

De préférence encore, après cette mise en contact, l'utilisateur referme la matrice 3 de manière à recouvrir, de préférence totalement, le comprimé 9.

Après déballage complet, le comprimé ainsi conditionné peut être donné à l'animal.

Comme cela apparaît clairement, le conditionnement d'une substance active au moyen d'un support selon l'invention est très simple et peut être mis en oeuvre extemporanément, à l'unité, par le particulier lui-même. Il ne nécessite aucun appareillage.

Avantageusement, le même procédé de conditionnement et les mêmes supports 1 peuvent être utilisés pour des substances actives de formes très différentes.

Avantageusement, l'utilisateur peut conditionner plusieurs substances actives, se présentant éventuellement sous des formes différentes (gélule, comprimé, etc.) au moyen d'un même support 1.

On sait que certains animaux restent marqués par la première expérience d'un produit nouveau. Ainsi, lorsqu'on propose un médicament pour la première fois à un chien, il est possible de le tromper en enrobant un médicament au goût désagréable au moyen d'une matrice appétente. Mais si, après avoir mâché la matrice appétente, le chien ressent ce goût désagréable, il refusera par la suite d'avaler le même produit présenté de la même manière. En revanche, si le chien ne ressent pas le goût désagréable la première fois qu'on lui propose le médicament enrobé, il continuera à accepter ce médicament par la suite, même si, les fois suivantes, il ressent le goût désagréable.

Avantageusement, selon l'invention, il est possible de donner un premier support 1 sans substance active afin de faire accepter ce support par l'animal. Comme expliqué ci-dessus, cette étape préliminaire facilitera l'acceptation ultérieure des supports suivants contenant une substance active.

Le tableau 1 suivant démontre l'efficacité d'un support selon l'invention et l'intérêt particulier d'un enrobage au moyen d'un exhausteur d'appétence du type poudre de foie.

**TABLEAU 1**

| | % d'acceptation pour un chien |
|---|---|
| Support : cube de guimauve neutre | 25 % des cas (4 cas) |
| Support : cube de guimauve enrobé d'une poudre de foie | 90,5% des cas (21 cas) |

Le mode de réalisation décrit et représenté est fourni à titre d'exemple illustratif.

Par exemple, le support selon l'invention peut présenter une surface collante 7 orientée vers l'extérieur et recouverte, jusqu'à utilisation du support 1, d'un film protecteur, par exemple d'un film en matière plastique ou d'un papier gras, prévu pour n'adhérer que légèrement à la surface collante 7.

Par "légèrement" on entend que le film protecteur peut être retiré par l'utilisateur sans dégradation structurelle du support 1 ni diminution de l'adhésivité de la surface collante 7.

En retirant le film protecteur, l'utilisateur découvre ainsi une surface collante 7 apte à fixer la substance active 9.

Par ailleurs, l'invention concerne également un support 1 appétent pour une substance active 9 destinée à un animal comportant une matrice 3 en un matériau comestible remarquable en ce que ladite matrice 3 est déformable de manière à permettre un assemblage de la substance active 9 et de la matrice 3.

Le collage de la substance active 9 sur la matrice 3 n'est alors plus indispensable.

De préférence, la déformabilité de la matrice 3 est plastique et l'assemblage résulte, au moins en partie, d'un recouvrement, au moins partiel, de la substance active 9 par la matrice 3 du support 1.

Il suffit dès lors de fermer suffisamment la matrice 3 autour de la substance active 9 pour la retenir.

De préférence encore, la matrice 3 peut être déformée à la main.

Les figures 1 à 7, dans lesquelles la surface 7 ne serait pas nécessairement collante, représentent des modes de réalisation possibles de tels supports déformables plastiquement.

L'assemblage peut également être assuré, ou amélioré, si la déformabilité du support 1 permet un enfermement permanent, au moins partiel, de la substance active 9 par un collage de deux extrémités 23 et 23' du support 1, comme représenté sur les figures 5, 6 et 7. De préférence, au moins une de ces extrémités 23 et 23' comporte une surface collante pouvant être apposée sur l'autre extrémité de manière à enfermer, au moins partiellement, la substance active 9.

De nouveau, à la différence des modes de réalisation décrits précédemment et illustrés sur les figures 5, 6 et 7, la surface de la matrice 3 en contact avec la substance active 9 n'est alors pas nécessairement collante.

De préférence, la matrice 3 est à la fois déformable plastiquement et comporte au moins deux extrémités, 23 et 23', pouvant être collées l'une sur l'autre.

Le collage des deux extrémités 23 et 23' l'une sur l'autre, combiné au caractère plastique de la matrice 3, permet de retenir très efficacement la substance active 9.

De préférence encore, la surface de la matrice 3 en contact avec la substance active 9 est également collante. Cette propriété est indispensable si le support 1 n'est pas déformable ou s'il est déformable mais sans permettre un assemblage de la substance active 9 avec la matrice 3.

Le caractère collant de différents supports a été évalué par deux tests. Pour chacun de ces tests, chaque support testé présentait une surface collante plane de 3 cm² et un poids de 2 grammes.

Les supports suivants ont été testés :
- la guimauve décrite ci-dessus, et
- un support appètent appelé Easypill® dont la composition est décrite ci-dessus.

A titre de comparaison, les supports suivants, hors invention, ont également été testés :
- un morceau de viande maigre de volaille, et
- un support à base de pâte carnée appelé Tab pocket®, commercialisé par la société Waltham et destiné aux chiens et aux chats.

Dans le premier test, la surface collante a été appliquée sur une plaque de verre, puis légèrement pressée sur cette plaque avec une force de 1 N. La plaque de verre a été retournée de manière que le support testé soit sous la plaque et subisse ainsi, de par son poids, une force d'arrachement. On a mesuré le temps entre le retournement de la plaque et la chute du support.

Les résultats du premier test sont résumés dans le tableau 1 suivant :

**Tableau 1**

| Support testé | Temps observé entre le retournement de la plaque de verre et la chute du support |
|---|---|
| Guimauve | > 3 minutes |
| Easypill® | > 3 minutes |
| Tab pocket® | 0 |
| Viande | < 30 secondes |

Dans un deuxième test, le support est fixé sur la plaque de verre comme dans le premier test, une force de 1 N étant appliquée sur le support pour presser la surface collante sur la plaque de verre. La plaque de verre est ensuite maintenue en position horizontale, le support à tester étant au-dessus de la plaque de verre. On mesure alors la force de traction nécessaire pour désolidariser le support de la plaque de verre.

Les résultats du deuxième test sont résumés dans le tableau 2 suivant :

**Tableau 2**

| Support | Force appliquée pour observer la désolidarisation entre la plaque de verre et le support testé |
|---|---|
| Guimauve | 0,3 N |
| Easypill® | 0,15 N |
| Tab pocket® | Entre 0,020 N et 0,025 N |
| Viande | Entre 0,020 N et 0,025 N |

Les surfaces collantes 7 de la guimauve et de Easypill® ont pu retenir pendant plus de trois minutes les granulés suivants, collés sous le support de manière à subir une force d'arrachement du fait de la gravité : un comprimé de CUREPAR CANIN® commercialisé par le laboratoire SOGEVAL (masse : 0,5 g ; diamètre: 0,1cm; épaisseur: 0,6cm), un comprimé de ENACARD® 15-30 kg, commercialisé par le laboratoire MERIAL (masse : 0,2g ; diamètre : 0,8cm ; épaisseur : 0,4cm), un comprimé de CANIDIARIX® commercialisé par le laboratoire TVM (masse : 0,4g ; diamètre : 1cm ; épaisseur : 0,55cm), ou encore une gélule de Flore Process®, commercialisée par le laboratoire ORIGINAL PROCESS (masse : 0,3g ; longueur : 1,9cm ; diamètre : 0,6cm).

## Revendications

1. Support appétent (1) pour une substance active destinée à un animal, comportant une matrice (3) en un matériau comestible, la matrice (3) comprenant au moins une surface collante (7) destinée à fixer ladite substance active, **caractérisé en ce que** ladite matrice (3) est :
- soit en une matière choisie parmi une pâte de viande, d'abats ou de viscères, incorporant au moins 10% en poids d'au moins un agent collant, ledit agent collant étant un sirop de glucose,
- soit en une guimauve comprenant du sucre ou l'un de ses substituts, du sirop de glucose et de la gélatine,
et **en ce que** la surface collante (7) est suffisamment adhésive pour que la substance active (9) ne puisse être détachée de la matrice (3) sans que la matrice (3) laisse un dépôt de matière sur la substance active (9).

2. Support selon la revendication 1, **caractérisé en ce que** la quantité dudit agent collant est supérieure à 25% en poids par rapport au poids total de ladite matrice (3).

3. Support selon la revendication 1 ou la revendication 2, **caractérisé en ce que** ladite matrice (3) comporte au moins deux surfaces (34, 36) collées l'une à l'autre et conformées de manière à pouvoir être écartées l'une de l'autre, à la main, découvrant ainsi ladite surface collante (7).

4. Support selon l'une quelconque des revendications précédentes, **caractérisé en ce que** ladite matrice (3) comporte au moins une masse gluante pouvant être ouverte de manière à exposer ladite surface collante (7).

5. Support selon la revendication 4, **caractérisé en ce que** ladite matrice (3) est déchirable à la main de manière à ouvrir ladite masse gluante.

6. Support selon l'une quelconque des revendications précédentes, **caractérisé en ce que** ladite matrice (3) est déformable à la main.

7. Support selon l'une quelconque des revendications précédentes, **caractérisé en ce que** ladite matrice (3) renferme une substance appétente.

8. Support selon l'une quelconque des revendications précédentes, **caractérisé en ce qu'**il comporte une couche (5) d'une substance appétente recouvrant, au moins en partie, ladite matrice (3).

9. Support selon la revendication 7 ou la revendication 8, **caractérisé en ce que** ladite substance appétente comporte une poudre de foie.

10. Support selon l'une quelconque des revendications précédentes, **caractérisé en ce que** ladite matrice (3) est en une matière appétente pour ledit animal.

11. Procédé de conditionnement d'une substance active (9) destinée à un animal au moyen d'un support appétent (1) selon l'une des revendications 1 à 10, **caractérisé en ce qu'**il comporte une étape de collage de ladite substance active (9) sur une surface collante (7) de ladite matrice (3).

12. Procédé selon la revendication 11, **caractérisé en ce que**, préalablement audit collage, on ouvre ladite matrice (3) de manière à exposer ladite surface collante (7) vers l'extérieur (33) dudit support (1).

13. Procédé selon la revendication 12, **caractérisé en ce qu'**on procède à ladite ouverture par déchirement de ladite matrice (3).

14. Procédé selon l'une quelconque des revendications 11 à 13, **caractérisé en ce que**, après ledit collage, on referme ladite matrice (3) de manière à recouvrir, au moins partiellement, ladite substance active (9).

## Patentansprüche

1. Appetitanregender Träger (1) für einen für ein Tier bestimmten Wirkstoff, umfassend eine Matrix (3) aus einem verzehrbaren Material, wobei die Matrix (3) wenigstens eine klebende Oberfläche (7) aufweist, die dazu bestimmt ist, den Wirkstoff zu befestigen,
**dadurch gekennzeichnet, dass** die Matrix (3)
- entweder aus einem Stoff besteht, der aus einer Paste aus Fleisch, Schlachtabfällen oder Innereien ausgewählt ist und wenigstens 10 Gew.-% von wenigstens einem Klebrigmacher enthält, wobei es sich bei dem Klebrigmacher um einen Glukosesirup handelt,
- oder aus einer Schaumzuckerware besteht, die Zucker oder einen seiner Ersatzstoffe, Glukosesirup und Gelatine enthält,
und dass die klebende Oberfläche (7) ausreichend haftend ist, so dass der Wirkstoff (9) nicht von der Matrix (3) gelöst werden kann, ohne dass die Matrix (3) eine Stoffablagerung auf dem Wirkstoff (9) zurücklässt.

2. Träger nach Anspruch 1, **dadurch gekennzeichnet, dass** die Menge des Klebrigmachers über 25 Gew.-% bezogen auf das Gesamtgewicht der Matrix (3) beträgt.

3. Träger nach Anspruch 1 oder Anspruch 2, **dadurch gekennzeichnet, dass** die Matrix (3) wenigstens zwei Oberflächen (34, 36) umfasst, die aneinandergeklebt sind und derart geformt sind, dass sie mit der Hand voneinander entfernt werden können, wodurch die klebende Oberfläche (7) offengelegt wird.

4. Träger nach einem der vorhergehenden Ansprüche, **dadurch gekennzeichnet, dass** die Matrix (3) wenigstens eine klebrige Masse umfasst, die geöffnet werden kann, um die klebende Oberfläche (7) freizulegen.

5. Träger nach Anspruch 4, **dadurch gekennzeichnet, dass** die Matrix (3) mit der Hand zerrissen werden kann, um die klebrige Masse zu öffnen.

6. Träger nach einem der vorhergehenden Ansprüche, **dadurch gekennzeichnet, dass** die Matrix (3) mit der Hand verformt werden kann.

7. Träger nach einem der vorhergehenden Ansprüche, **dadurch gekennzeichnet, dass** die Matrix (3) eine appetitanregende Substanz einschließt.

8. Träger nach einem der vorhergehenden Ansprüche, **dadurch gekennzeichnet, dass** er eine Schicht (5) aus einer appetitanregenden Substanz umfasst, welche die Matrix (3) wenigstens teilweise bedeckt.

9. Träger nach Anspruch 7 oder Anspruch 8, **dadurch gekennzeichnet, dass** die appetitanregende Substanz ein Leberpulver umfasst.

10. Träger nach einem der vorhergehenden Ansprüche, **dadurch gekennzeichnet, dass** die Matrix (3) aus einem Stoff besteht, der für das Tier appetitanregend ist.

11. Verfahren zur Verpackung eines für ein Tier bestimmten Wirkstoffs (9) mithilfe eines appetitanregenden Trägers (1) nach einem der Ansprüche 1 bis 10,
**dadurch gekennzeichnet, dass** es einen Schritt des Klebens des Wirkstoffs (9) auf eine klebende Oberfläche (7) der Matrix (3) umfasst.

12. Verfahren nach Anspruch 11, **dadurch gekennzeichnet, dass** vor dem Kleben die Matrix (3) geöffnet wird, um die klebende Oberfläche (7) zum Äußeren (33) des Trägers (1) hin freizulegen.

13. Verfahren nach Anspruch 12, **dadurch gekennzeichnet, dass** die Öffnung durch Zerreißen der Matrix (3) erfolgt.

14. Verfahren nach einem der Ansprüche 11 bis 13, **dadurch gekennzeichnet, dass** nach dem Kleben die Matrix (3) geschlossen wird, um den Wirkstoff (9) wenigstens teilweise zu bedecken.

## Claims

1. Palatable carrier (1) for an active substance intended for an animal, comprising a matrix (3) of an edible material, the matrix (3) having at least one tacky surface (7) for fixing said active substance,
**characterised in that** said matrix (3) is:
- either made of a material selected from a meat, offal or visceral paste, incorporating at least 10% by weight of at least one tackifying agent, said tackifying agent being glucose syrup,
- or made of a marshmallow comprising sugar or a sugar substitute, glucose syrup and gelatin,
and **in that** the tacky surface (7) is sufficiently adhesive that the active substance (9) cannot be detached from the matrix (3) without the matrix (3) leaving a deposit of material on the active substance (9).

2. Carrier according to claim 1, **characterised in that** the quantity of said tackifying agent is greater than 25% by weight relative to the total weight of said matrix (3).

3. Carrier according to claim 1 or claim 2, **characterised in that** said matrix (3) has at least two surfaces (34, 36) which are stuck to one another and are so configured that they can be separated from one another, by hand, thus uncovering said tacky surface (7).

4. Carrier according to any one of the preceding claims, **characterised in that** said matrix (3) comprises at least one sticky mass which can be opened so as to expose said tacky surface (7).

5. Carrier according to claim 4, **characterised in that** said matrix (3) can be torn by hand so as to open said sticky mass.

6. Carrier according to any one of the preceding claims, **characterised in that** said matrix (3) can be moulded by hand.

7. Carrier according to any one of the preceding claims, **characterised in that** said matrix (3) contains a palatable substance.

8. Carrier according to any one of the preceding claims, **characterised in that** it comprises a layer (5) of a palatable substance covering, at least in part, said matrix (3).

9. Carrier according to claim 7 or claim 8, **characterised in that** said palatable substance comprises a liver powder.

10. Carrier according to any one of the preceding claims, **characterised in that** said matrix (3) is made of a material which is palatable to said animal.

11. Method of encasing an active substance (9) intended for an animal by means of a palatable carrier (1) according to any one of claims 1 to 10, **characterised in that** it comprises a step of sticking said active substance (9) to a tacky surface (7) of said matrix (3).

12. Method according to claim 11, **characterised in that**, prior to said sticking, said matrix (3) is opened so as to expose said tacky surface (7) to the outside (33) of said carrier (1).

13. Method according to claim 12, **characterised in that** said opening is effected by tearing said matrix (3).

14. Method according to any one of claims 11 to 13, **characterised in that**, after said sticking, said matrix (3) is closed so as to cover, at least partially, said active substance (9).
